# EUROPEAN PATENT APPLICATION

(11) **EP 1 741 722 A2**
(43) Date of publication of application: **10.01.2007**
(21) Application number: 06014233.8
(22) Date of filing: 17.10.2001
(51) Int. Cl.: C07K 14/705, C12Q 1/68

(54) **Polymorphisms in the human P2X7 gene**

(30) Priority: 21.10.2000 GB 0025859; 02.11.2000 US 244897 P; 06.04.2001 GB 0108654
(62) Divisional of application: 01308837.2
(71) Applicant: AstraZeneca AB, 151 85 Södertälje (SE)
(72) Inventor: Morten, John Edward Norris, Macclesfield Cheshire, SK10 4TG (GB)

(57) **Abstract**

This invention relates to polymorphisms in the human P2X₇ gene and corresponding novel allelic polypeptides encoded thereby. The invention also relates to methods and materials for analysing allelic variation in the P2X₇ gene, and to the use of P2X₇ polymorphism in treatment of diseases with P2X₇ drugs.

## Description

This invention relates to polymorphisms in the human P2X₇ gene and corresponding novel allelic polypeptides encoded thereby. The invention also relates to methods and materials for analysing allelic variation in the P2X₇ gene, and to the use of P2X₇ polymorphism in treatment of diseases with P2X₇ drugs.

The P2X₇ receptor (previously known as P2Z receptor), which is a ligand-gated ion channel, is present on a variety of cell types, largely those known to be involved in the inflammatory/immune process, specifically, macrophages, mast cells and lymphocytes (T and B). Activation of the P2X₇ receptor by extracellular nucleotides, in particular adenosine triphosphate, leads to the release of interleukin-1β (IL-1β) and giant cell formation (macrophages/microglial cells), degranulation (mast cells) and L-selectin shedding (lymphocytes). P2X₇ receptors are also located on antigen-presenting cells (APC), keratinocytes, salivary acinar cells (parotid cells) and hepatocytes. Compounds acting at the P2X₇ receptor are therefore indicated as pharmaceuticals for use in the treatment of rheumatoid arthritis, osteoarthritis, psoriasis, allergic dermatitis, asthma, chronic obstructive pulmonary disease (COPD), hyperresponsiveness of the airway, septic shock, glomerulonephritis, irritable bowel disease, Crohn's disease, ulcerative colitis, atherosclerosis, growth and metastases of malignant cells, myoblastic leukaemia, diabetes, Alzheimer's disease, meningitis, osteoporosis, bum injury, ischaemic heart disease, stroke and varicose veins. For further background, the reader is referred to the following articles: North and Barnard in Current Opinion in Neurobiology 1997, 7, 346-357; Rassendren, JBC, 1997, 273, 5482-6; and Buell, Receptors and Channels, 1998, 5, 347-354. The terms P2X₇, P2X₇ receptor and P2RX7 are used interchangeably herein.

All positions herein of polymorphisms in the 5' UTR region of the P2X₇ polynucleotide relate to the position in SEQ ID NO 1 unless stated otherwise or apparent from the context.

All positions herein of polymorphisms in the exon regions of the P2X₇ polynucleotide relate to the position in SEQ ID NO 2 unless stated otherwise or apparent from the context.

All positions herein of polymorphisms in the intron regions of the P2X₇ polynucleotide relate to the position in SEQ ID NO 3 unless stated otherwise or apparent from the context.

All positions herein of polymorphisms in the P2X₇ polypeptide relate to the position in SEQ ID NO 4 unless stated otherwise or apparent from the context.

One approach is to use knowledge of polymorphisms to help identify patients most suited to therapy with particular pharmaceutical agents (this is often termed "pharmacogenetics") . Pharmacogenetics can also be used in pharmaceutical research to assist the drug selection process. Polymorphisms are used in mapping the human genome and to elucidate the genetic component of diseases. The reader is directed to the following references for background details on pharmacogenetics and other uses of polymorphism detection: Linder et al. (1997), Clinical Chemistry, 43, 254; Marshall (1997), Nature Biotechnology, 15, 1249; International Patent Application WO 97/40462, Spectra Biomedical; and Schafer et al. (1998), Nature Biotechnology, 16, 33.

Clinical trials have shown that patient response to treatment with pharmaceuticals is often heterogeneous. Thus there is a need for improved approaches to pharmaceutical agent design and therapy.

Point mutations in polypeptides will be referred to as follows: natural amino acid (using 1 or 3 letter nomenclature), position, new amino acid. For (a hypothetical) example "D25K" or "Asp25Lys" means that at position 25 an aspartic acid (D) has been changed to lysine (K). Multiple mutations in one polypeptide will be shown between square brackets with individual mutations separated by commas.

The present invention is based on the discovery of polymorphisms in P2X₇. In particular, we have found thirty polymorphisms in the coding sequence of the P2X₇ gene, 12 of which lead to changes in the sequence of expressed protein.

According to one aspect of the present invention there is provided a method for the diagnosis of a polymorphism in P2X₇ in a human, which method comprises determining the sequence of the human at at least one polymorphic position and determining the status of the human by reference to polymorphism in P2X₇. Preferred polymorphic positions are one or more of the following positions:
positions 936, 1012, 1147, 1343 and 1476 in the 5'UTR region of the P2X₇ gene as defined by the position in SEQ ID NO: 1;
positions 253, 488, 489, 760, 835, 853, 1068, 1096, 1315, 1324, 1405, 1448, 1494, 1513, 1628 and 1772 in the coding region of the P2X₇ gene as defined by the position in SEQ ID NO: 2; and
positions 4780, 4845, 4849, 5021, 5554, 5579, 5535, 5845 and 6911 in the intron region of the P2X₇ gene as defined by the position in SEQ ID NO: 3;
positions 76 ,155, 245, 270, 276, 348, 357, 430, 433, 460, 490 and 496 in the P2X7 polypeptide as defined by the position in SEQ ID NO: 4.

The term human includes both a human having or suspected of having a P2X₇ mediated disease and an asymptomatic human who may be tested for predisposition or susceptibility to such disease. At each position the human may be homozygous for an allele or the human may be a heterozygote.

The term "status" refers to the genetic status of the human as detected by potential sequence variation at defined positions of a polynucleotide or corresponding protein. The term "diagnosis of a polymorphism" refers to determination of the genetic status of an individual at a polymorphic position (in which the indiviual may be homozygous or heterozygous at each position).

The term polymorphism includes single nucleotide substitution, nucleotide insertion and nucleotide deletion which in the case of insertion and deletion includes insertion or deletion of one or more nucleotides at a position of a gene and corresponding alterations in expressed protein.

In one embodiment of the invention preferably the method for diagnosis described herein is one in which the polymorphism in the in the 5'UTR region of the P2X₇ gene as defined by the position in SEQ ID NO: 1 is any one of the following:
at position 936 is presence of C and/or A; at position 1012 is presence of T and/or C;
at position 1147 is presence of A and/or G; at position 1343 is presence of G and/or A; and
at position 1476 is presence of A and/or G.

In one embodiment of the invention preferably the method for diagnosis described herein is one in which the polymorphism in the coding region of the P2X₇ gene as defined by the position in SEQ ID NO: 2 is any one of the following:
at position 253 is presence of T and/or C; at position 488 is presence of G and/or A;
at position 489 is presence of C and/or T; at position 760 is presence of T and/or G;
at position 835 is presence of G and/or A; at position 853 is presence of G and/or A;
at position 1068 is presence of G and/or A; at position 1096 is presence of C and/or G;
at position 1315 is presence of C and/or G; at position 1324 is presence of C and/or T;
at position 1405 is presence of A and/or G; at position 1448 is presence of C and/or T;
at position 1494 is presence of A and/or G; at position 1513 is presence of A and/or C;
at position 1628 is presence of G and/or T; and at position 1772 is presence of G and/or A.

In one embodiment of the invention preferably the method for diagnosis described herein is one in which the polymorphism in the intron region of the P2X₇ gene as defined by the position in SEQ ID NO: 3. is any one of the following:
at position 4780 is presence of C and/or T; at position 4845 is presence of C and/or T;
at position 4849 is presence of A and/or C; at position 5021 is presence of T and/or C;
at position 5554 is presence of 3 and/or 4 repeats of GTTT (wherein position 5554 refers to the position of the G in the first unit repeat);
at position 5579 is presence of G and/or C; at position 5535 is presence of A and/or T;
at position 5845 is presence of C and/or T; and at position 6911 is presence of T and/or C.

In one embodiment of the invention preferably the method for diagnosis described herein is one in which the polymorphism in the P2X₇ protein as defined by the position in SEQ ID NO: 4. is any one of the following: va176ala, his155tyr, va1245gly, arg270his, arg276his, ala348thr, thr357ser, pro430arg, ala433val, gln460arg, ser490gly and glu496ala.

The method for diagnosis is preferably one in which the sequence is determined by a method selected from amplification refractory mutation system, restriction fragment length polymorphism and primer extension.

The status of the individual may be determined by reference to allelic variation at any 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more positions.

The test sample of nucleic acid is conveniently a sample of blood, bronchoalveolar lavage fluid, sputum, or other body fluid or tissue obtained from an individual. It will be appreciated that the test sample may equally be a nucleic acid sequence corresponding to the sequence in the test sample, that is to say that all or a part of the region in the sample nucleic acid may firstly be amplified using any convenient technique e.g. PCR, before analysis of allelic variation.

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 1 lists a number of mutation detection techniques, some based on the PCR. These may be used in combination with a number of signal generation systems, a selection of which is listed in Table 2. Further amplification techniques are listed in Table 3. Many current methods for the detection of allelic variation are reviewed by Nollau et al., Clin. Chem. 43, 1114-1120, 1997; and in standard textbooks, for example "Laboratory Protocols for Mutation Detection", Ed. by U. Landegren, Oxford University Press, 1996 and "PCR", 2nd Edition by Newton & Graham, BIOS Scientific Publishers Limited, 1997.

### Abbreviations:

| | |
|---|---|
| ALEX™ | Amplification refractory mutation system linear extension |
| APEX | Arrayed primer extension |
| ARMS™ | Amplification refractory mutation system |
| b-DNA | Branched DNA |
| bp | base pair |
| CMC | Chemical mismatch cleavage |
| COPS | Competitive oligonucleotide priming system |
| DGGE | Denaturing gradient gel electrophoresis |
| ELISA | Enzyme Linked ImmunoSorbent Assay |
| FRET | Fluorescence resonance energy transfer |
| LCR | Ligase chain reaction |
| MASDA | Multiple allele specific diagnostic assay |
| NASBA | Nucleic acid sequence based amplification |
| OLA | Oligonucleotide ligation assay |
| PCR | Polymerase chain reaction |
| PTT | Protein truncation test |
| RFLP | Restriction fragment length polymorphism |
| SDA | Strand displacement amplification |
| SNP | Single nucleotide polymorphism |
| SSCP | Single-strand conformation polymorphism analysis |
| SSR | Self sustained replication |
| TGGE | Temperature gradient gel electrophoresis |

### Table 1 - Mutation Detection Techniques

### General: DNA sequencing, Sequencing by hybridisation

**Scanning:** PTT*, SSCP, DGGE, TGGE, Cleavase, Heteroduplex analysis, CMC, Enzymatic mismatch cleavage
* Note: not useful for detection of promoter polymorphisms.

### Hybridisation Based

Solid phase hybridisation: Dot blots, MASDA, Reverse dot blots, Oligonucleotide arrays (DNA Chips).

Solution phase hybridisation: Taqman™ - US-5210015 & US-5487972 (Hoffmann-La Roche), Molecular Beacons - Tyagi et al (1996), Nature Biotechnology, 14, 303; WO 95/13399 (Public Health Inst., New York)
**Extension Based:** ARMS™, ALEX™ - European Patent No. EP 332435 B1 (Zeneca Limited), COPS - Gibbs et al (1989), Nucleic Acids Research, 17, 2347.
**Incorporation Based:** Mini-sequencing, APEX
**Restriction Enzyme Based:** RFLP, Restriction site generating PCR
**Ligation Based**: OLA
**Other:** Invader assay

### Table 2 - Signal Generation or Detection Systems

**Fluorescence:** FRET, Fluorescence quenching, Fluorescence polarisation - United Kingdom Patent No. 2228998 (Zeneca Limited)
**Other:** Chemiluminescence, Electrochemiluminescence, Raman, Radioactivity, Colorimetric, Hybridisation protection assay, Mass spectrometry

### Table 3 - Further Amplification Methods

SSR, NASBA, LCR, SDA, b-DNA

### Table 4- Protein variation detection methods

### Immunoassay

### Immunohistology

### Peptide sequencing

Preferred mutation detection techniques include ARMS™, ALEX™, COPS, Taqman, Molecular Beacons, RFLP, and restriction site based PCR and FRET techniques. Immunoassay techniques are known in the art e.g. A Practical Guide to ELISA by D M Kemeny, Pergamon Press 1991; Principles and Practice of Immunoassay, 2nd edition, C P Price & D J Newman, 1997, published by Stockton Press in USA & Canada and by Macmillan Reference in the United Kingdom.. Histological techniques are described in Theory and Practice of Histological Techniques by J D Bancroft & A Stevens, 4th Edition, Churchill Livingstone,1996. Protein sequencing is described in Laboratory Techniques in Biochemistry and Molecular Biology, Volume 9, Sequencing of Proteins and Peptides, G Allen, 2nd revised edition, Elsevier, 1989. Particularly preferred methods include ARMS™ and RFLP based methods. ARMS™ is an especially preferred method.

In a further aspect, the diagnostic methods of the invention are used to assess the pharmacogenetics of a drug acting at P2X₇.

Assays, for example reporter-based assays, may be devised to detect whether one or more of the above polymorphisms affect transcription levels and/or message stability.

Individuals who carry particular allelic variants of the P2X₇ gene may therefore exhibit differences in their ability to regulate protein biosynthesis under different physiological conditions and will display altered abilities to react to different diseases. In addition, differences arising as a result of allelic variation may have a direct effect on the response of an individual to drug therapy. The diagnostic methods of the invention may be useful both to predict the clinical response to such agents and to determine therapeutic dose.

In a further aspect, the diagnostic methods of the invention, are used to assess the predisposition and/or susceptibility of an individual to diseases mediated by P2X₇. This may be particularly relevant in the development of hyperlipoproteinemia and cardiovascular disease and the present invention may be used to recognise individuals who are particularly at risk from developing these conditions.

In a further aspect, the diagnostic methods of the invention are used in the development of new drug therapies which selectively target one or more allelic variants of the P2X₇ gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes.

According to another aspect of the present invention there is provided a human P2X₇ gene or its complementary strand comprising a variant allelic polymorphism at one or more of positions defined herein or a fragment thereof of at least 20 bases comprising at least one novel polymorphism.

Fragments are at least 17 bases, more preferably at least 20 bases, more preferably at least 30 bases.

According to another aspect of the present invention there is provided a polynucleotide comprising at least 20 bases of the human P2X₇ gene and comprising a polymorphism selected from any one of the following:

| **Region** | **Polymorphism** |
|---|---|
| | **SEQ ID NO: 1** |
| 5' UTR | 936 C→A |
| | 1012 T→C |
| | 1147 A→G |
| | 1343 G→A |
| | 1476 A→G |

| **Region** | **Polymorphism** |
|---|---|
| | **SEQ ID NO: 2** |
| exon 2 | 253 T→C |
| exon 5 | 488 G→A |
| | 489 C→T |
| exon 7 | 760 T→G |
| exon 8 | 835 G→A |
| | 853 G→A |
| exon 11 | 1068 G→A |
| | 1096 C→G |
| exon 12 | 1315 C→G |
| exon 13 | 1324 C→T |
| | 1405 A→G |
| | 1448 C→T |
| | 1494 A→G |
| | 1513 A→C |
| | 1628 G→T |
| | 1772 G→A |

| **Region** | **Polymorphism** |
|---|---|
| | **SEQ ID NO: 3** |
| intron E | 4780 C→T |
| | 4845 C→T |
| | 4849 A→C |
| intron F | 5021 T→C |
| | 5554 (GTTT)n=3,4 |
| | 5579 G→C |
| | 5535 A→T |
| intron G | 5845 C→T |
| | 6911 T→C |

According to another aspect of the present invention there is provided a polynucleotide comprising at least 20 bases of the human P2X₇ gene and comprising an allelic variant selected from any one of the following:

| **Region** | **Variant** |
|---|---|
| | **SEQ ID NO: 1** |
| 5' UTR | 936 A |
| | 1012 C |
| | 1147 G |
| | 1343 A |
| | 1476 G |

| **Region** | **Variant** |
|---|---|
| | **SEQ ID NO: 2** |
| exon 2 | 253 C |
| exon 5 | 488 A |
| | 489 T |
| exon 7 | 760 G |
| exon 8 | 835 A |
| | 853 A |
| exon 11 | 1068 A |
| | 1096 G |
| exon 12 | 1315 G |
| exon 13 | 1324 T |
| | 1405 G |
| | 1448 T |
| | 1494 G |
| | 1513 C |
| | 1628 T |
| | 1772 A |

| **Region** | **Variant** |
|---|---|
| | **SEQ ID NO: 3** |
| intron E | 4780 T |
| | 4845 T |
| | 4849 C |
| intron F | 5021 C |
| | 5554 (GTTT)ₙ,n=4 |
| | 5579 C |
| | 5535 T |
| intron G | 5845 T |
| | 6911 C |

According to another aspect of the present invention there is provided a human P2X₇ gene or its complementary strand comprising a polymorphism, preferably corresponding with one or more the positions defined herein or a fragment thereof of at least 20 bases comprising at least one polymorphism.

Fragments are at least 17 bases, more preferably at least 20 bases, more preferably at least 30 bases.

The invention further provides a nucleotide primer which can detect a polymorphism of the invention.

According to another aspect of the present invention there is provided an allele specific primer capable of detecting a P2X₇ gene polymorphism, preferably at one or more of the positions as defined herein.

An allele specific primer is used, generally together with a constant primer, in an amplification reaction such as a PCR reaction, which provides the discrimination between alleles through selective amplification of one allele at a particular sequence position e.g. as used for ARMS™ assays. The allele specific primer is preferably 17- 50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer.

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1st Edition. If required the primer(s) may be labelled to facilitate detection.

According to another aspect of the present invention there is provided an allele-specific oligonucleotide probe capable of detecting a P2X₇ gene polymorphism, preferably at one or more of the positions defined herein.

The allele-specific oligonucleotide probe is preferably 17- 50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. The probes of the invention may carry one or more labels to facilitate detection.

According to another aspect of the present invention there is provided an allele specific primer or an allele specific oligonucleotide probe capable of detecting a P2X₇ gene polymorphism at one of the positions defined herein.

According to another aspect of the present invention there is provided a diagnostic kit comprising an allele specific oligonucleotide probe of the invention and/or an allele-specific primer of the invention.

The diagnostic kits may comprise appropriate packaging and instructions for use in the methods of the invention. Such kits may further comprise appropriate buffer(s) and polymerase(s) such as thermostable polymerases, for example taq polymerase.

In another aspect of the invention, the polymorphisms of this invention may be used as genetic markers in linkage studies. This particularly applies to the polymorphisms of relatively high frequency. The P2X₇ gene is on chromosome 12q24 (Buell et al, Receptors and Channels, 1998, 5,347-354). Low frequency polymorphisms may be particularly useful for haplotyping as described below. A haplotype is a set of alleles found at linked polymorphic sites (such as within a gene) on a single (paternal or maternal) chromosome. If recombination within the gene is random, there may be as many as 2ⁿ haplotypes, where 2 is the number of alleles at each SNP and n is the number of SNPs. One approach to identifying mutations or polymorphisms which are correlated with clinical response is to carry out an association study using all the haplotypes that can be identified in the population of interest. The frequency of each haplotype is limited by the frequency of its rarest allele, so that SNPs with low frequency alleles are particularly useful as markers of low frequency haplotypes. As particular mutations or polymorphisms associated with certain clinical features, such as adverse or abnormal events, are likely to be of low frequency within the population, low frequency SNPs may be particularly useful in identifying these mutations (for examples see:
Linkage disequilibrium at the cystathionine beta synthase (CBS) locus and the association between genetic variation at the CBS locus and plasma levels of homocysteine. Ann Hum Genet (1998) 62:481-90, De Stefano V, Dekou V, Nicaud V, Chasse JF, London J, Stansbie D, Humphries SE, and Gudnason V; and Variation at the von willebrand factor (vWF) gene locus is associated with plasma vWF:Ag levels: identification of three novel single nucleotide polymorphisms in the vWF gene promoter. Blood (1999) 93:4277-83, Keightley AM, Lam YM, Brady JN, Cameron CL, Lillicrap D).

According to another aspect of the present invention there is provided a computer readable medium comprising at least one novel sequence of the invention stored on the medium. The computer readable medium may be used, for example, in homology searching, mapping, haplotyping, genotyping or pharmacogenetic analysis.

According to another aspect of the present invention there is provided a method of treating a human in need of treatment with a drug acting at P2X₇ in which the method comprises:
i) diagnosis of a polymorphism in P2X₇ in the human, which diagnosis preferably comprises determining the sequence at one or more of the following positions:
   positions 936, 1012, 1147, 1343 and 1476 in the 5'UTR region of the P2X₇ gene as defined by the position in SEQ ID NO: 1;
   positions 253, 488, 489, 760, 835, 853, 1068, 1096, 1315, 1324, 1405, 1448, 1494, 1513, 1628 and 1772 in the coding region of the P2X₇ gene as defined by the position in SEQ ID NO: 2; and
   positions 4780, 4845, 4849, 5021, 5554, 5579, 5535, 5845 and 6911 in the intron region of the P2X₇ gene as defined by the position in SEQ ID NO: 3; and
   positions 76 ,155, 245, 270, 276, 348, 357, 430, 433, 460, 490 and 496 in the P2X₇ polypeptide as defined by the position in SEQ ID NO: 4;
   and determining the status of the human by reference to polymorphism in P2X₇; and
ii) administering an effective amount of the drug.

Preferably determination of the status of the human is clinically useful. Examples of clinical usefulness include deciding which drug or drugs to administer and/or in deciding on the effective amount of the drug or drugs. The term "drug acting at P2X7" means that drug binding with P2X7 in humans is an important part of a drug exerting its pharmceutical effect in man. Compounds which are known to be antagonists of the P2X7 receptor are described in published PCT application nos. WO 99/29660, WO 99/29661, WO 99/29686, WO 00/61569, WO 00/71529, WO 01/42194, WO 01/44170, WO 01/44213 and WO 01/46200.

According to another aspect of the present invention there is provided use of a drug acting at P2X₇ in preparation of a medicament for treating a disease in a human diagnosed as having a polymorphism therein, preferably at one or more of the positions defined herein.

According to another aspect of the present invention there is provided a pharmaceutical pack comprising P2X₇ drug and instructions for administration of the drug to humans diagnostically tested for a polymorphism therein, preferably at one or more of the positions defined herein.

According to another aspect of the present invention there is provided an allelic variant of human P2X₇ polypeptide comprising at least one of the following:
a alanine at position 76 of SEQ ID NO 4;
a tyrosine at position 155 of SEQ ID NO 4;
a glycine at position 245 of SEQ ID NO 4;
a histidine at position 270 of SEQ ID NO 4;
a histidine at position 276 of SEQ ID NO 4;
a threonine at position 348 of SEQ ID NO 4;
a serine at position 357 of SEQ ID NO 4;
a arginine at position 430 of SEQ ID NO 4;
a valine at position 433 of SEQ ID NO 4;
a arginine at position 460 of SEQ ID NO 4;
a glycine at position 490 of SEQ ID NO 4; and
a glutamic acid at position 496 of SEQ ID NO 4;
or a fragment thereof comprising at least 10 amino acids provided that the fragment comprises at least one allelic variant.

Fragments of polypeptide are at least 10 amino acids, more preferably at least 15 amino acids, more preferably at least 20 amino acids.

According to another aspect of the present invention there is provided an antibody specific for an allelic variant of human P2X₇ polypeptide as described herein.

Antibodies can be prepared using any suitable method. For example, purified polypeptide may be utilized to prepare specific antibodies. The term "antibodies" is meant to include polycional antibodies, monoclonal antibodies, and the various types of antibody constructs such as for example F(ab')₂, Fab and single chain Fv. Antibodies are defined to be specifically binding if they bind the allelic variant of P2X₇ with a Kₐ of greater than or equal to about 10⁷ M⁻¹. Affinity of binding can be determined using conventional techniques, for example those described by Scatchard et al., Ann. N.Y. Acad. Sci., 51:660 (1949).

Polyclonal antibodies can be readily generated from a variety of sources, for example, horses, cows, goats, sheep, dogs, chickens, rabbits, mice or rats, using procedures that are well-known in the art. In general, antigen is administered to the host animal typically through parenteral injection. The immunogenicity of antigen may be enhanced through the use of an adjuvant, for example, Freund's complete or incomplete adjuvant. Following booster immunizations, small samples of serum are collected and tested for reactivity to antigen. Examples of various assays useful for such determination include those described in:
Antibodies: A Laboratory Manual, Harlow and Lane (eds.), Cold Spring Harbor Laboratory Press, 1988; as well as procedures such as countercurrent immuno-electrophoresis (CIEP), radioimmunoassay, radioimmunoprecipitation, enzyme-linked immuno-sorbent assays (ELISA), dot blot assays, and sandwich assays, see U.S. Patent Nos. 4,376,110 and 4,486,530.

Monoclonal antibodies may be readily prepared using well-known procedures, see for example, the procedures described in U.S. Patent Nos. RE 32,011, 4,902,614, 4,543,439 and 4,411,993; Monoclonal Antibodies, Hybridomas: A New Dimension in Biological Analyses, Plenum Press, Kennett, McKearn, and Bechtol (eds.), (1980).

The monoclonal antibodies of the invention can be produced using alternative techniques, such as those described by Alting-Mees et al., "Monoclonal Antibody Expression Libraries: A Rapid Alternative to Hybridomas", Strategies in Molecular Biology 3: 1-9 (1990) which is incorporated herein by reference. Similarly, binding partners can be constructed using recombinant DNA techniques to incorporate the variable regions of a gene that encodes a specific binding antibody. Such a technique is described in Larrick et al., Biotechnology, 7: 394 (1989).

Once isolated and purified, the antibodies may be used to detect the presence of antigen in a sample using established assay protocols, see for example "A Practical Guide to ELISA" by D. M. Kemeny, Pergamon Press, Oxford, England.

According to another aspect of the invention there is provided a diagnostic kit comprising an antibody of the invention.

According to another aspect of the present invention there is provided a polynucleotide comprising any one of the following twenty six P2X₇ haplotypes:

| | **1012** | **489** | **5579** | **835** | **853** | **1068** | **1096** | **1405** | **1513** |
|---|---|---|---|---|---|---|---|---|---|
| | SEQ ID 1 | SEQ ID 2 | SEQ ID 3 | SEQ ID 2 | SEQ ID 2 | SEQ ID 2 | SEQ ID 2 | SEQ ID 2 | SEQ ID 2 |
| ***1*** | T | T | C | G | G | A | G | A | A |
| ***2*** | C | C | G | G | G | G | C | A | A |
| ***3*** | C | C | C | A | G | G | C | A | C |
| ***4*** | C | T | G | G | G | A | C | G | A |
| ***5*** | C | C | G | G | G | A | G | A | A |
| ***6*** | C | C | C | A | G | G | C | A | A |
| ***7*** | T | T | G | G | G | A | C | G | A |
| ***8*** | C | T | C | G | G | G | C | A | A |
| ***9*** | C | C | C | G | G | A | C | A | A |
| ***10*** | C | T | G | G | G | G | C | A | C |
| ***11*** | T | C | G | G | G | A | C | A | A |
| ***12*** | C | T | C | G | G | G | C | A | C |
| ***13*** | T | C | C | G | G | A | C | A | A |
| ***14*** | T | C | C | G | G | G | C | A | C |
| ***15*** | C | T | C | G | G | A | C | A | A |
| ***16*** | T | T | C | G | G | A | C | G | A |
| ***17*** | C | C | G | G | G | A | C | G | A |
| ***18*** | T | C | G | A | A | G | C | A | A |
| ***19*** | C | C | C | G | G | G | G | A | A |
| ***20*** | T | C | C | G | G | G | G | A | A |
| ***21*** | C | T | C | A | G | G | C | A | A |
| ***22*** | C | C | C | G | G | G | C | A | C |
| ***23*** | C | T | G | G | A | A | G | G | A |
| ***24*** | T | T | G | G | G | A | G | G | A |
| ***25*** | C | T | C | G | G | G | G | A | A |
| ***26*** | C | C | C | G | G | G | C | A | A |

According to another aspect of the present invention there is provided a human P2X₇ polypeptide comprising one of the following eighteen combinations of alleleic variant determined amino acids based on positions identified in SEQ ID NO: 4:

| | **155** | **270** | **276** | **348** | **357** | **460** | **496** |
|---|---|---|---|---|---|---|---|
| ***1*** | Y | R | R | T | S | Q | E |
| ***2*** | Y | R | R | T | T | R | E |
| ***3*** | Y | R | R | T | T | Q | E |
| ***4*** | Y | R | R | T | S | R | E |
| ***5*** | Y | R | R | A | T | Q | A |
| ***6*** | Y | R | R | A | T | Q | E |
| ***7*** | Y | R | R | A | S | Q | E |
| ***8*** | Y | R | H | T | S | R | E |
| ***9*** | Y | H | R | A | T | Q | E |
| ***10*** | H | R | R | T | T | Q | E |
| ***11*** | H | R | R | T | T | R | E |
| ***12*** | H | R | R | A | T | Q | A |
| ***13*** | H | R | R | A | S | Q | E |
| ***14*** | H | R | R | A | T | Q | E |
| ***15*** | H | R | R | T | S | Q | E |
| ***16*** | H | H | R | A | T | Q | A |
| ***17*** | H | H | R | A | T | Q | E |
| ***18*** | H | H | H | A | T | Q | E |

According to another aspect of the present invention there is provided a polynucleotide which encodes any human P2X₇ polypeptide combination of allelic variants defined herein.

The invention will now be illustrated but not limited by reference to the following Examples. All temperatures are in degrees Celsius.

In the Examples below, unless otherwise stated, the following methodology and materials have been applied.

AMPLITAQ™ ,available from Perkin-Elmer Cetus, is used as the source of thermostable DNA polymerase.

General molecular biology procedures can be followed from any of the methods described in "Molecular Cloning - A Laboratory Manual" Second Edition, Sambrook, Fritsch and Maniatis (Cold Spring Harbor Laboratory, 1989) or "Current Protocols in Molecular Biology", Volumes1-3, Edited by F M Asubel, R Brent & R E Kingston, published by John Wiley, 1998.

Electropherograms were obtained in a standard manner: data was collected by ABI377 data collection software and the wave form generated by ABI Prism sequencing analysis (2.1.2).

### Example 1

### Identification of Polymorphisms

### 1. Methods

### DNA Preparation

DNA was prepared from frozen blood samples collected in EDTA following protocol I (Molecular Cloning: A Laboratory Manual, p392, Sambrook, Fritsch and Maniatis, 2nd Edition, Cold Spring Harbor Press, 1989) with the following modifications. The thawed blood was diluted in an equal volume of standard saline citrate instead of phosphate buffered saline to remove lysed red blood cells. Samples were extracted with phenol, then phenol/chloroform and then chloroform rather than with three phenol extractions. The DNA was dissolved in deionised water.

### Template Preparation

Templates were prepared by PCR using the oligonucleotide primers and annealing temperatures set out below. The extension temperature was 72° and denaturation temperature 94°. Generally 50 ng of genomic DNA was used in each reaction and subjected to 35 cycles of PCR. Where described below, the primary fragment was diluted 1/100 and two microlitres were used as template for amplification of secondary fragments. PCR was performed in two stages (primary fragment then secondary fragment) to ensure specific amplification of the desired target sequence.

### Polymorphisms in P2X₇

| | | | | |
|---|---|---|---|---|
| **Region** | **Size** | **Polymorphism** | **protein change** | **frequency** |
| 5' UTR | | 936 C→A | | 3/56 |
| | | 1012 T→C | | 42/56 |
| | | 1147 A→G | | 3/56 |
| | | 1343 G→A | | 2/52 |
| | | 1476 A→G | | 35/52 |
| exon 1 | 146bp | | | |
| intron A | 21.7kb | | | |
| exon 2 | 168bp | 253 T→C | va176a1a | 2/54 |
| intron B | 1.1kb | | | |
| exon 3 | 68bp | | | |
| intron C | 4.7kb | | | |
| exon 4 | 73bp | | | |
| intron D | 1.5kb | | | |
| exon 5 | 95bp | 488 G→A | silent | 2/54 |
| | | 489 C→T | his155tyr | 17/38 |
| intron E | 2.8kb | 4780 C→T | | 39/52 |
| | | 4845 C→T | | 39/52 |
| | | 4849 A→C | | 28/36 |
| exon 6 | 80bp | | | |
| intron F | 617bp | 5021 T→C | | 1/34 |
| | | 5554 (GTTT) n=3,4 | | n=3, 14/40 |
| | | 5579 G→C | | 26/40 |
| | | 5535 A→T | | 1/44 |
| exon 7 | 129bp | 760 T→G | va1245gly | 1/40 |
| intron G | 1.3kb | 5845 C→T | | 2/40 |
| | | 6911 T→C | | 33/50 |
| exon 8 | 136bp | 835 G→A | arg270his | 16/52 |
| | | 853 G→A | arg276his | 1/54 |
| intron H | | | | |
| exon 9 | 91bp | | | |
| intron I | 1.7kb | | | |
| exon 10 | 64bp | | | |
| intron J | 84bp | | | |
| exon 11 | 149bp | 1068 G→A | ala348thr | 18/62 |
| | | 1096 C→G | thr357ser | 5/66 |
| intron K | | | | |
| exon 12 | 101bp | 1315 C→G | pro430arg, splice site | 4/66 |
| intron L | 3.8kb | | | |
| exon 13 | 497bp | 1324 C→T | ala433val | 1/54 |
| | | 1405 A→G | gln460arg | 3/54 |
| | | 1448 C→T | silent | 2/54 |
| | | 1494 A→G | ser490gly | 2/54 |
| | | 1513 A→C | glu496ala | 8/54 |
| | | 1628 G→T | silent | 2/52 |
| | | 1772 G→A | silent | 24/54 |

Positions in the 5' UTR refer to SEQ ID NO: 1.
Positions in exons refer to SEQ ID NO: 2.
Positions in introns refer to SEQ ID NO: 3.
Positions in protein refer to SEQ ID NO: 4.

Evidence for effects of some polymorphisms on transcription are as follows. C at position 1012 SEQ ID No 1 disrupts the TCAAT motif from an enhancer binding sequence reported in intron 1 of EGFR. A at position 1147 SEQID No 1 disrupts the reverse sequenc of the TCCTGC motif which is also an enhancer binding sequence from intron 1 EGFR. (Maekawa T., Imamoto F., Merlino G. T., Pastan I., Ishii S. Cooperative Function of Two Separate Enhancers of RT the Human Epidermal Growth Factor Receptor Proto-oncogene J. Biol. Chem. 264:5488-5494 (1989)).

### 15 Example 2

### Haplotype analysis

### a) The following allele frequencies were determined in a Swedish population.

| **SEQ ID NO** | **Position** | **Frequency** |
|---|---|---|
| 1 | 1012 | 46/60 |
| 2 | 489 | 27/60 |
| 3 | 5579 | 39/60 |
| 2 | 835 | 16/58 |
| 2 | 853 | 3/60 |
| 2 | 1068 | 24/58 |
| 2 | 1096 | 6/58 |
| 2 | 1045 | 11/60 |
| 2 | 1513 | 10/60 |

### b) Haplotype data.

Analysis of 15 Swedish families with at least one asthmatic child using primer extension (SNapShot™, Perkin Elmer) genotyping and GeneHunter™ analysis demonstrate the following haplotypes:

| | **1012** | **489** | **5579** | **835** | **853** | **1068** | **1096** | **1405** | **1513** | **Frequency n/58** |
|---|---|---|---|---|---|---|---|---|---|---|
| ***1*** | T | T | C | G | G | A | G | A | A | **1** |
| ***2*** | C | C | G | G | G | G | C | A | A | **3** |
| ***3*** | C | C | C | A | G | G | C | A | C | **1** |
| ***9*** | C | T | G | G | G | A | C | G | A | **5** |
| ***5*** | C | C | G | G | G | A | G | A | A | **1** |
| ***6*** | C | C | C | A | G | G | C | A | A | **8** |
| ***7*** | T | T | G | G | G | A | C | G | A | **1** |
| ***8*** | C | T | C | G | G | G | C | A | A | **3** |
| ***9*** | C | C | C | G | G | A | C | A | A | **3** |
| ***10*** | C | T | G | G | G | G | C | A | C | **2** |
| ***11*** | T | C | G | G | G | A | C | A | A | **2** |
| ***12*** | C | T | C | G | G | G | C | A | C | **3** |
| ***13*** | T | C | C | G | G | A | C | A | A | **4** |
| ***14*** | T | C | C | G | G | G | C | A | C | **1** |
| ***15*** | C | T | C | G | G | A | C | A | A | **2** |
| ***16*** | T | T | C | G | G | A | C | G | A | **1** |
| ***17*** | C | C | G | G | G | A | C | G | A | **2** |
| ***18*** | T | C | G | A | A | G | C | A | A | **2** |
| ***19*** | C | C | C | G | G | G | G | A | A | **1** |
| ***20*** | T | C | C | G | G | G | G | A | A | **1** |
| ***21*** | C | T | C | A | G | G | C | A | A | **4** |
| ***22*** | C | C | C | G | G | G | C | A | C | **3** |
| ***23*** | C | T | G | G | A | A | G | G | A | **1** |
| ***24*** | T | T | G | G | G | A | G | G | A | **1** |
| ***25*** | C | T | C | G | G | G | G | A | A | **1** |
| ***26*** | C | C | C | G | G | G | C | A | A | **1** |

This results in the following proteins:

| ***position SEQ ID NO 4*** | **155** | **270** | **276** | **348** | **357** | **460** | **496** | **Frequency N/58** |
|---|---|---|---|---|---|---|---|---|
| ***amino acid*** | Y | R | R | T | S | Q | E | 1 |
| | Y | R | R | T | T | R | E | 7 |
| | Y | R | R | T | T | Q | E | **2** |
| | Y | R | R | T | S | R | E | **1** |
| | Y | R | R | A | T | Q | A | **5** |
| | Y | R | R | A | T | Q | E | **3** |
| | Y | R | R | A | S | Q | E | **1** |
| | Y | R | H | T | S | R | E | **1** |
| | Y | H | R | A | T | Q | E | **4** |
| | H | R | R | T | T | Q | E | **9** |
| | H | R | R | T | T | R | E | **2** |
| | H | R | R | A | T | Q | A | **4** |
| | H | R | R | A | S | Q | E | **3** |
| | H | R | R | A | T | Q | E | **3** |
| | H | R | R | T | S | Q | E | **1** |
| | H | H | R | A | T | Q | A | **1** |
| | H | H | R | A | T | Q | E | **8** |
| | H | H | H | A | T | Q | E | **2** |

### c) Analysis

Ben J. Gu, Weiyi Zhang, Rebecca A. Worthington, Ronald Sluyter, Phuong Dao-Ung, Steven Petrou, Julian A. Barden, and James S. Wiley, J. Biol. Chem. (2001) 276: 11135-11142 reported that Ala at 496 (C at 1513) leads to loss of function in P2X7. Only one polymorphism was reported since they only analysed the final exon for SNPs

## Claims

1. A method for the diagnosis of a polymorphism in P2X₇ in a human, which method comprises determining the sequence of the human at one or more of the following positions:
positions 936, 1012, 1147, 1343 and 1476 in the 5'UTR region of the P2X₇ gene as defined by the position in SEQ ID NO: 1;
positions 253, 488, 489, 760, 835, 853, 1068, 1096, 1315, 1324, 1405, 1448, 1494, 1513, 1628 and 1772 in the coding region of the P2X₇ gene as defined by the position in SEQ ID NO: 2; and
positions 4780, 4845, 4849, 5021, 5554, 5579, 5535, 5845 and 6911 in the intron region of the P2X₇ gene as defined by the position in SEQ ID NO: 3;
positions 76 ,155, 245, 270, 276, 348, 357, 430, 433, 460, 490 and 496 in the P2X₇ polypeptide as defined by the position in SEQ ID NO: 4;
and determining the status of the human by reference to polymorphism in P2X₇.

2. Use of a diagnostic method as defined in claim 1 to assess the pharmacogenetics of a drug acting at P2X₇.

3. A polynucleotide comprising at least 20 bases of the human P2X₇ gene and comprising an allelic variant selected from any one of the following:
| **Region** | **Variant** |
|---|---|
| | **SEQ ID NO: 1** |
| 5'UTR | 936 A |
| | 1012 C |
| | 1147 G |
| | 1343 A |
| | 1476 G |
| **Region** | **Variant** |
|---|---|
| | **SEQ ID NO: 2** |
| exon 2 | 253 C |
| exon 5 | 488 A |
| | 489 T |
| exon 7 | 760 G |
| exon 8 | 835 A |
| | 853 A |
| exon 11 | 1068 A |
| | 1096 G |
| exon 12 | 1315 G |
| exon 13 | 1324 T |
| | 1405 G |
| | 1448 T |
| | 1494 G |
| | 1513 C |
| | 1628 T |
| | 1772 A |
| **Region** | **Variant** |
|---|---|
| | **SEQ ID NO: 3** |
| intron E | 4780 T |
| | 4845 T |
| | 4849 C |
| intron F | 5021 C |
| | 5554 (GTTT)ₙ,n=4 |
| | 5579 C |
| | 5535 T |
| intron G | 5845 T |
| | 6911 C |

4. A nucleotide primer which can detect a polymorphism as defined in claim 1.

5. An allele specific primer capable of detecting a P2X₇ gene polymorphism as defined in claim 1.

6. An allele-specific oligonucleotide probe capable of detecting a P2X₇ gene polymorphism as defined in claim 1.

7. Use of a P2X₇ gene polymorphism as defined in claim 1 as a genetic marker in a linkage study.

8. A method of treating a human in need of treatment with a drug acting at P2X₇ in which the method comprises:
i) diagnosis of a polymorphism in P2X₇ in the human, which diagnosis preferably comprises determining the sequence at one or more of the following positions:
positions 936, 1012, 1147, 1343 and 1476 in the 5'UTR region of the P2X₇ gene as defined by the position in SEQ ID NO: 1;
positions 253, 488, 489, 760, 835, 853, 1068, 1096, 1315, 1324, 1405, 1448, 1494, 1513, 1628 and 1772 in the coding region of the P2X₇ gene as defined by the position in SEQ ID NO: 2; and
positions 4780, 4845, 4849, 5021, 5554, 5579, 5535, 5845 and 6911 in the intron region of the P2X₇ gene as defined by the position in SEQ ID NO: 3; and
positions 76 ,155, 245, 270, 276, 348, 357, 430, 433, 460, 490 and 496 in the P2X₇ polypeptide as defined by the position in SEQ ID NO: 4;
and determining the status of the human by reference to polymorphism in P2X₇ ; and
ii) administering an effective amount of the drug.

9. An allelic variant of human P2X₇ polypeptide comprising at least one of the following:
a alanine at position 76 of SEQ ID NO 4;
a tyrosine at position 155 of SEQ ID NO 4;
a glycine at position 245 of SEQ ID NO 4;
a histidine at position 270 of SEQ ID NO 4;
a histidine at position 276 of SEQ ID NO 4;
a threonine at position 348 of SEQ ID NO 4;
a serine at position 357 of SEQ ID NO 4;
a arginine at position 430 of SEQ ID NO 4;
a valine at position 433 of SEQ ID NO 4;
a arginine at position 460 of SEQ ID NO 4;
a glycine at position 490 of SEQ ID NO 4; and
a glutamic acid at position 496 of SEQ ID NO 4;
or a fragment thereof comprising at least 10 amino acids provided that the fragment comprises at least one allelic variant.

10. An antibody specific for an allelic variant of human P2X₇ polypeptide as defined in claim 9.

11. A polynucleotide comprising any one of the following twenty six P2X₇ haplotypes:
| | **1012** | **489** | **5579** | **835** | **853** | **1068** | **1096** | **1405** | **1513** |
|---|---|---|---|---|---|---|---|---|---|
| | SEQ ID 1 | SEQ ID 2 | SEQ ID 3 | SEQ ID 2 | SEQ ID 2 | SEQ ID 2 | SEQ ID 2 | SEQ ID 2 | SEQ ID 2 |
| ***1*** | T | T | C | G | G | A | G | A | A |
| ***2*** | C | C | G | G | G | G | C | A | A |
| ***3*** | C | C | C | A | G | G | C | A | C |
| ***4*** | C | T | G | G | G | A | G | G | A |
| ***5*** | C | C | G | G | G | A | G | A | A |
| ***6*** | C | C | C | A | G | G | C | A | A |
| ***7*** | T | T | G | G | G | A | C | G | A |
| ***8*** | C | T | C | G | G | G | C | A | A |
| ***9*** | C | C | C | G | G | A | C | A | A |
| ***10*** | C | T | G | G | G | G | C | A | C |
| ***11*** | T | C | G | G | G | A | C | A | A |
| ***12*** | C | T | C | G | G | G | C | A | C |
| ***13*** | T | C | C | G | G | A | C | A | A |
| ***14*** | T | C | C | G | G | G | C | A | C |
| ***15*** | C | T | C | G | G | A | C | A | A |
| ***16*** | T | T | C | G | G | A | C | G | A |
| ***17*** | C | C | G | G | G | A | C | G | A |
| ***18*** | T | C | G | A | A | G | C | A | A |
| ***19*** | C | C | C | G | G | G | G | A | A |
| ***20*** | T | C | C | G | G | G | G | A | A |
| ***21*** | C | T | C | A | G | G | C | A | A |
| ***22*** | C | C | C | G | G | G | C | A | C |
| ***23*** | C | T | G | G | A | A | G | G | A |
| ***24*** | T | T | G | G | G | A | G | G | A |
| ***25*** | C | T | C | G | G | G | G | A | A |
| ***26*** | C | C | C | G | G | G | C | A | A |

12. A human P2X₇ polypeptide comprising one of the following eighteen combinations of alleleic variant determined amino acids based on positions identified in SEQ ID NO: 4:
| | **155** | **270** | **276** | **348** | **357** | **460** | **496** |
|---|---|---|---|---|---|---|---|
| ***1*** | Y | R | R | T | S | Q | E |
| ***2*** | Y | R | R | T | T | R | E |
| ***3*** | Y | R | R | T | T | Q | E |
| ***4*** | Y | R | R | T | S | R | E |
| ***5*** | Y | R | R | A | T | Q | A |
| ***6*** | Y | R | R | A | T | Q | E |
| ***7*** | Y | R | R | A | S | Q | E |
| ***8*** | Y | R | H | T | S | R | E |
| ***9*** | Y | H | R | A | T | Q | E |
| ***10*** | H | R | R | T | T | Q | E |
| ***11*** | H | R | R | T | T | R | E |
| ***12*** | H | R | R | A | T | Q | A |
| ***13*** | H | R | R | A | S | Q | E |
| ***14*** | H | R | R | A | T | Q | E |
| ***15*** | H | R | R | T | S | Q | E |
| ***16*** | H | H | R | A | T | Q | A |
| ***17*** | H | H | R | A | T | Q | E |
| ***18*** | H | H | H | A | T | Q | E |

13. A polynucleotide which encodes any human P2X₇ polypeptide as defined in claim 12.


SEQUENCE LISTING

<110> AstraZeneca AB

<120> Chemical Compounds

<130> morten

<140>
<141>

<160> 4

<170> PatentIn Ver. 2.1

<210> 1
<211> 4900
<212> DNA
<213> Homo sapiens

<400> 1
gactcactat agggagaccg gcagatctga tatcatcgcc actgtggatc cgaattctag 60
aaggcctatg ttctaagcat caggctttac ctgtgaatct cctcttttta cagatgaaga 120
tgactgtatc actcagattc ccggcaggaa agcaatggca tactcaagtg gggtaactaa 180
tgatggaacc atttacaaag gtgtggacag agttaagaaa aagcaatagg agatagtgag 240
cttcctgggg ctggtaagag tggggagccc ttaccactcc caggactaaa ggagggagtg 300
gtgcccagaa gccctgccta tatgcaactg agaagggcag ggccagggag tcacgtccat 360
cctcactgct ctccagtctc ctgaactgga agccagaagg tgaggggaac cctgatgcag 420
tttgtatgtg tgagaaagta caattagttt agactgaaaa actgaaaatc tacccggcca 480
cttagcaggc tggaataaca gaaatggatc aagccagctg taaagataac agggaacaat 540
aattctctgt agctgtaaag tgataataca accctgcatc tttgagtgac tgctgaaaca 600
ttgtccttta aaatcagaga ccttcagaaa cttcgctgtt tgaaattaca tgactaagac 660
tgaaatattc caattttgcc tggaagattt aagtcatctt gacacagaga agcagcctca 720
atttacaact caggagcaga gcttcagata aagattttct ggacacattt gacatgtatc 780
ttagctatgt tgcttcctag gaaacagggc cctgggtcct ctttgcaatc cagactgaag 840
ttgactgctt tgtacaaacc tgttttgctt tgagtccatc aaaacatgac ttcatttaga 900
ttttatctca actccacttt cctcggaatc ctatactaaa ttgctgtttt cctttgtttg 960
gtgatgtgcg tagctcttct ggtgggtggt gtccctcact gaataggtca ataaacctaa 1020
ctttgttgga ctgccactgt gtccctggtg atctttggct gattggtcta ggtcatagat 1080
cgacctgccg gggtgcagag gagggtggag agtaactcag agggtcaagc atgaaagatc 1140
tggcagaaaa ataaagcccc tccaccccca ccacccctac ccctgcaaat ctgatttccc 1200
ccaccaactg cagaccagag tattataagg ggcggtggaa gaagaggggg agatcttcat 1260
ttacccagag ctcctataca tcaggggctg aataaagggt tgtagaaatg aatgaatcaa 1320
tctctgagtg gggcttcagg cagtggaaag atctcagtcc ttttctgagg cataatggaa 1380
gctcccagtc ttgtgacatt tgcaaggctg cccctttctc ccaagagaca tgagaccaaa 1440
aaagtgaaag gaaagggggg aaaagggaga attctaaaaa tgcccatcct ctgaacacca 1500
tctttgtgta ggcatctggg ggaggccagc tggggtgagg tcatctgcca gccaggcccg 1560
taggacttgg cgcttcttgt ttatcacagc cacatgtggg gccactgcca gggcccgccc 1620

caactctgca gtcattggag gagcttgaag ttaaagactc ctgctaaaaa ccagtacgtt 1680
tcattttgca gttactggga gggggcttgc tgtggccctg tcaggaagag tagagctctg 1740
gtccagctcc gcgcagggag ggaggctgtc accatgccgg cctgctgcag ctgcagtgat 1800
gttttccagt atgagacgaa caaagtcact cggatccaga gcatgaatta tggcaccatt 1860
aagtggttct tccacgtgat catcttttcc tacgtttggt aagtgggatc tggggaggac 1920
ccagatctct gcagtggccg acagcacaga aagccccagc gggcagcttc aggtgcacat 1980
tctgaatctc acatggtttt cgaatctgag acgtgctctc acagccagct gggcgggagg 2040
gaggaagcag cagcaggcaa gaggaaacgg tgccaggctg cagcagagag aagccacagg 2100
acaagcggga ttcctttctg ctctacttca ggcccgccag ggcgcgcaag gcagggcgtg 2160
cctggggaag gtaggaaagc gcagggcaac accctggatc cccagggagg aggcgaggat 2220
ctcagggcac gcctggtgat catgctggca tctgagtcac catgcttggg aggaatagga 2280
ccaggcttga aaatgtgtta taactttagg tcctcaccaa cgtcaggaag gccctgcttt 2340
ttggtttttg tttcttctaa aagaaactta ctgagatata atttatacac catacaattg 2400
acccatttaa agggtaccat ttaatgattt tcagattatt cccagagttg tgcaaccatc 2460
cccacaatca attttagaat attttaatcg actcaaaagg aatcccacac tccttcacca 2520
tcatttccaa cactgttcct cctccttccc acccatcaat ttcctttctg cctctatgga 2580
tttgccgatt ctggacattt catataaatg gaatcacata atatgtggtc ctttgtggca 2640
cttagcgtgt tttcaatgct catccatgtt gtagcatgtg ttgatacttc attcaatttt 2700
tttttttaaa gagacggggt ctcactattt tgtccaggct ggtctcaaac tcctggactc 2760
aagtgatctg cctgcctcgg cctcccaaag tgtcaggatt acaggcgtga gccattgcac 2820
ccggctgata cttcattcct ttttacggct gagtagtact ccattgcatg gatagaccac 2880
ttttttctat ccattcatcc attgatggac attggggttg tttctctttt ttggctatca 2940
tgaataatgc cacatgaaca tttgtgtaca aggttttatg tggatatata ttctcctttc 3000
tctcgaatat gtacctaaga gtaaaaattg ctaggtcata tgttaactat gtttcacctt 3060
tggggggaat gtggagctga atttcacagc agctgcagtt ttttacattc ctatcagcag 3120
agtatgaggg atccaatttc tccacatcct caccaacgct tgttatcgtc tgtctttttg 3180
ggttttgttg ttgtcatttt gtttttgtct ttgagatgaa gtcttgctct gttgcccagg 3240
ctggagtgca gtggcgcaat cttggctcac tgcaacctcc acctccccgg ttcaagcgat 3300
tctcctgcct cagcctcatg agtagctggg attacaggtg tgcgtcacca ctcctcacta 3360
atttttgtat ttttagtaga gatgaggttt cgccatgtag gccaggctgg tctcaaactc 3420
ctgacctcaa gtgatccgcc caccttggcc tcccaaagtg ctgggattac aggcatgagc 3480
cgccacgccc ggctgattgt ctgtcttttt tattatagcc atgctagtgg gtgtgaagtg 3540
gtagttcatt gtggttgtga tttgaatttc cctgatggtg agtgcctctt attctctgtg 3600
ctgattgata atgatgatga aggcaatttg tatctataga gtggcagtgt agtttactaa 3660
gagttagggt aacttatttc atagtactgg ctatgtcttc tgggccaagt cattaacttc 3720
tctgagcctc agtttctgca tctgttcata gggttgtggc aattaaccaa aaaaaaaagg 3780
catgaacagc ccttatcatg atgactgaca taggataaga gctccataac tagtatctat 3840
ttttaaaaat aatcttttta agtctgggag tggtggctca cacctgtaat cccaacactt 3900
tgggaggccg aggcgggtgg atcacgaagt caggagtttg agaccagcct ggccaatatg 3960
gtgaaacccc atctctacta aaaatacaaa aattagtggg gagtggtggt gcacacctgt 4020
aatcccagct actagggagg ctgaggcagg agaatcgctt gaacccggag gcggaggttg 4080
cagtgagccg agatcaagcc actgcactcc agcctgggtg acagagcaag actccatctc 4140
aaaataataa taatagtaat aatttttttg attatataat agtatatatg tatataaaat 4200
acatgtatgt atttttatct atatcctctg ctctgaccct caaagtaacc acgtccaagt 4260
tcaggatttg aaatctggaa acgtggattc aaaaatcctt cacctctttg agccttggtt 4320
tcatcatctg taaaatgggg agaattgttg ataggaatat taaatgaact aataaatgca 4380
aagctgtttg agaaatatat ggcatatagt aatccctgat taagtgttag ttcttattat 4440
taataatgct attattagga ttattattat tcgattcata tgtttactgt tcaacaaata 4500

ttgaatgata aacatatatg ctgggtccgg catggtggcc catgcctgta attccagcac 4560
tttgggaggc caaggcgggc aggtcacttg aggtcaagag tttgagacca gcctggccaa 4620
tgtggtggaa actccatctg tgctaaaaat acaaaaatta gccgggcatg gtggtgggtg 4680
cctgtaatcc cagctactcg ggaggctgag acaggagaat cacttgaacc caggaggtgg 4740
aggttgcagt gagccaagat tgcaccactg cactccagcc tgagccacag agcaagactc 4800
tgtctcaaaa aaaaaaaaaa aaaatatata tatatatata tatatatata gtatttttag 4860
tagagatggg gttttgccat ctcttatata tttttatatt 4900

<210> 2
<211> 1853
<212> DNA
<213> Homo sapiens

<400> 2
   aaaacgcagg gagggaggct gtcaccatgc cggcctgctg cagctgcagt gatgttttcc 60
   agtatgagac gaacaaagtc actcggatcc agagcatgaa ttatggcacc attaagtggt 120
   tcttccacgt gatcatcttt tcctacgttt gctttgctct ggtgagtgac aagctgtacc 180
   agcggaaaga gcctgtcatc agttctgtgc acaccaaggt gaaggggata gcagaggtga 240
   aagaggagat cgtggagaat ggagtgaaga agttggtgca cagtgtcttt gacaccgcag 300
   actacacctt ccctttgcag gggaactctt tcttcgtgat gacaaacttt ctcaaaacag 360
   aaggccaaga gcagcggttg tgtcccgagt atcccacccg caggacgctc tgttcctctg 420
   accgaggttg taaaaaggga tggatggacc cgcagagcaa aggaattcag accggaaggt 480
   gtgtagtgca tgaagggaac cagaagacct gtgaagtctc tgcctggtgc cccatcgagg 540
   cagtggaaga ggcccccggg cctgctctct tgaacagtgc cgaaaacttc actgtgctca 600
   tcaagaacaa tatcgacttc cccggccaca actacaccac gagaaacatc ctgccaggtt 660
   taaacatcac ttgtaccttc cacaagactc agaatccaca gtgtcccatt ttccgactag 720
   gagacatctt ccgagaaaca ggcgataatt tttcagatgt ggcaattcag ggcggaataa 780
   tgggcattga gatctactgg gactgcaacc tagaccgttg gttccatcac tgccgtccca 840
   aatacagttt ccgtcgcctt gacgacaaga ccaccaacgt gtccttgtac cctggctaca 900
   acttcagata cgccaagtac tacaaggaaa acaatgttga gaaacggact ctgataaaag 960
   tcttcgggat ccgttttgac atcctggttt ttggcaccgg aggaaaattt gacattatcc 1020
   agctggttgt gtacatcggc tcaaccctct cctacttcgg tctggccgct gtgttcatcg 1080
   acttcctcat cgacacttac tccagtaact gctgtcgctc ccatatttat ccctggtgca 1140
   agtgctgtca gccctgtgtg gtcaacgaat actactacag gaagaagtgc gagtccattg 1200
   tggagccaaa gccgacatta aagtatgtgt cctttgtgga tgaatcccac attaggatgg 1260
   tgaaccagca gctactaggg agaagtctgc aagatgtcaa gggccaagaa gtcccaagac 1320
   ctgcgatgga cttcacagat ttgtccaggc tgcccctggc cctccatgac acacccccga 1380
   ttcctggaca accagaggag atacagctgc ttagaaagga ggcgactcct agatccaggg 1440
   atagccccgt ctggtgccag tgtggaagct gcctcccatc tcaactccct gagagccaca 1500
   ggtgcctgga ggagctgtgc tgccggaaaa agccgggggc ctgcatcacc acctcagagc 1560
   tgttcaggaa gctggtcctg tccagacacg tcctgcagtt cctcctgctc taccaggagc 1620
   ccttgctggc gctggatgtg gattccacca acagccggct gcggcactgt gcctacaggt 1680
   gctacgccac ctggcgcttc ggctcccagg acatggctga ctttgccatc ctgcccagct 1740
   gctgccgctg gaggatccgg aaagagtttc cgaagagtga agggcagtac agtggcttca 1800
   agagtcctta ctgaagccag gcaccgtggc tcacgtctgt aatcccacct ttt 1853

<210> 3
<211> 11266
<212> DNA
<213> Homo sapiens

<400> 3
catcacctac aaaggaaacc ccaaatccag tagcagtcac tccccattct ccccttcccc 60
tgtccctggc cacagtctac tttctgtctc tatagatgcc tattctggac atttcctata 120
aatagaattg tatatggtgt ggccttttgt gtctgtcttc tttcactcag catcatgttc 180
tccaggtcca tccatgttgt agcctgtgtc attgcttcat ccttcttatg gctaaataag 240
attctgtgta tgaatgtacc acattttatt tgtccattca tccgtcagtg gccacttgca 300
tggtttccac ttttttggcg attctgagta gtgctgctat aagcattcgt gtgcacattc 360
tggtggatat cgaatcactt ctccacatct tagtaacaca cgtcacttac tccccactct 420
gtcatccttc tatctgcagt atcccacccg caggacgctc tgttcctctg accgaggttg 480
taaaaaggga tggatggacc cgcagagcaa aggtaccttc tgtttctttt cccgagaccc 540
taggggtgga tggtctggca tcttggtgac atttgtgatg cccaggtcag gtcttcagcc 600
tctgctctca gctgccctct tccaccatca ccaagccata ggcgagtctg cccatgcttc 660
ggctctgtcc ccagcagacc agctgctgac tgtaaacatg actccagttt tccagtgaga 720
gaagaagctc ctaaaaacct agcaggttca ggattctaat cggtagaaaa ttcacatggc 780
ctatagcatc atctgagtat tctaaacttt ccccctgaat ttcctcaaag gttgaggacc 840
atgaactttt acccccaggg gaacctggca gcaataccca tattaacctg cagaattttt 900
tttgtttttt attttatttt attttttaaa cattttttgc actgttttat tttgattttg 960
attttgattt tatttatatc taagtgcagt gctattgcga tactgcagaa tttctttatc 1020
tcacatttta acttaaaaag gcacagggca gcgagcgcag aggctggtgc ctgtaatccc 1080
agcactttgg gagggtgagg cagatggatg cttgaggtca ggggttcgag aacagcctgg 1140
aaaacatggt gaaaccccgt ctctactaaa aatacaaaaa tcagccagac atggtggcac 1200
acgcttataa tcccagctac ttgggaggct gagacgtgag aatcacttga acctggaagg 1260
cagaggttgc agtgagccaa gatcatgcca ctgcactcca gcatgggtga cagagcgaga 1320
cccctttaaa aaaaaaaaaa aaaggcacag ggcaatttta aaaatactgc aaatagtaaa 1380
aaaaaaaaaa tcagtggtta taatgcaaac acacacaaaa aggcatatgc ccattactgc 1440
attctactcc atactgtatg tgtatttgag ttagtataaa agttatttta acattgctca 1500
ctatttaatt aattctccct tggaaactga ttaatcatcc tggcactcca ggaagatgtg 1560
ccatgctgat ttcatggctt tgcacatcct gggcaggctg tgtacccctt gagggacttg 1620
tgcccctttg agaggccatg ttctagtcca tttatactaa gtgagagcat acacctgttc 1680
cgctcccctc atgggcacct tttcttataa agaaacaaaa gagccagcag aatccacagt 1740
ctttctgtgt tctctctgat ctttattatg ttttgcttgt ttgccttgcc ttgtgttcgt 1800
tgtggttagg atgggcttga tggaagctga agctgcgtgg gttggaaagc ctggtcaaag 1860
cctagtctct cgcccgggtt gagttaatga tgtccctcct ggagaacgtc ctctctgcag 1920
ttctttcaca tctgtggttc tacgatgctt tgacccctat aggaattcag accggaaggt 1980
gtgtagtgca tgaagggaac cagaagacct gtgaagtctc tgcctggtgc cccatcgagg 2040
cagtggaaga ggccccccgg tgagtcgcat ggggagacag acacagtggc cctcagcggc 2100
gaccagatga ggccttgccg aggctgcttg ggccttcccc tctcagcaca gccctgcaaa 2160
gtcctgggtc ctaccggctt ggggacccct gcgctctgga tgcactgctt ggcacaaact 2220
agtatctctg ggagggccat ggtggttggt aaactgttgt aacactcctg taccaactgg 2280
taaatagcta ctaccctgag catccttggg tgtccctggc cccttccttc ccccagatct 2340
tccagggtac ccccagaccc cctcctgtag tgccacagca ggatcccttc tgacttgtca 2400
gtgtccatac tgagtgatca aggataggaa ggaaggaggg agatggaaag gaaggacgaa 2460
gcgaggaaag agaaggggaa ggggaggaaa aagcaaaagg ggtgagggta aaagaggggg 2520

ggaaaggaag ttttctcaaa ttaaatgctt acaatgacat acagatttgg tggtcccttg 2580
tattgatgct tcgcttcaat acacaaagtc acaatgttaa atctcagaag ccacaagggc 2640
tgatgtattt cagcagagaa tagttagaaa gacctggatt caattcctag ctctaacacc 2700
attttgctgt gtgtccttgg gaaaatggct taacctctct gagtttcagt gtcctcacct 2760
gtaaaagcag aataataatt tcaccaactt catagggctg ttgtaaggat taaatgagat 2820
gatacttgta cagttattgt aaggtaagcc ccatgcatgc ctggcttaca cacacacaca 2880
cacacacaca cacacacacg cacacacaca cacacacaca atctacccct agaagtgtgg 2940
tggttctaga ccagcactgt ccaattgaac ttgatgcagt gatggaaatt tctgtatctg 3000
tgctgtccaa tagggcagct actaggtaca tgtggctatt gagtacatga aatgcgacta 3060
ctgaattttt gaaagagatg atagatgata gatagaaaga tagatagata gataaataga 3120
tagataatag atagatagac aggtagatag atagatagat agatagatag atagatagat 3180
agatagagtt ttgctatgtt gcccaggctg gttttgaact cctgggctca agcgatcctc 3240
ctgccttggc ctcccaaagt gctggggtta caggtttgag ccattgctcc cagcctgaat 3300
ttttaattaa atttaaattt aaatagccac acatgtctag tggctaccat attggacagc 3360
gcagttctag accgatgtga ttcaggatca ttccctcagc atcgtggggc aaagagaaaa 3420
ctgccccaag ctggcctgta gaaggctcag gcgaaggttt cccaatgccg ggatgggggg 3480
tgcgctcagc agcatcaccc cttatgattc tcaatcgcta atagctccac tcaggttcat 3540
ttctcggtca ggggcatttc tttgggaatc acccagctct gggagataca gcagcctcca 3600
ctcaggtagt ccttgttcaa gacaagcggc ccttgactga ctgcagtttc agttccagct 3660
ctgctatcaa ctcactcatt aaataaactg catctccagt gtgcctgcct ctgggctgga 3720
ttttgacgtg acctgggcaa gcaactccct gaacttcagt ttctcatata ttatatgaat 3780
tagctaagat ggttcgttta atcattcatt caacacatcc atcaccacgt agtaggtgtt 3840
agatatttat ttcatacgta actacgcata agagactttg ctaagtttta ggtaaaatac 3900
aagtcccaga tacggagcaa gtctcaacca ctgtacatac ctgaatgtgt aattacatca 3960
ctgtaagagg tgccacagta aatgccactg ggtcttgtgt tagtccattc tcacacaaag 4020
aactacctag ccaggtgcgg tggctcacgc ctgtaatccc aacactttgg caggctgagg 4080
caggcggatc acttgaggtc aggagtttga taccagcctg gcaaacatgg tgaaacccca 4140
tctctattaa aaaatgcata aattagccag gtgtggtggc acacgcctgt aatcctggct 4200
actcgggagg ctgaggcagg agagtcgctt gaacccggga gatggaggtt gcagtgaccc 4260
gagatcgcgc cactgcactc cagcctgggt gacagagtga gactccatct cagaaaaaaa 4320
taaaaataaa aataaagaac tacctgagac caaacacttt acgaaaaaaa agaggtttaa 4380
ttgactcaca gctccacagg cttaacagga agcctcagga gacttacaat catggcagaa 4440
ggcgaagggg aagcaaacac atcttaccat gatggagcag gaggcgggtt tcgggggatg 4500
tgccgcacac ttttaaatga tcagatctcg tgagaactca ctcactatca cgagaacagc 4560
aaggaggaag tccgccccca tgattcagtc acctcccacc aggcccctcc tctggcacat 4620
ggggattaca attcaagatg agatttgggt ggggacacag agccaaacca tatcagatct 4680
caagaaggga gaaattcttc ttggaggagc tggaggggct ttgtggagag tttcagaatg 4740
ctttgcccac taggtttgct gtatccattt ctcttcatgc atcccaaaga ccaagccaag 4800
aaaccagaag cctctggtcc cactggccca tgggctccct cggtccccac cgtcactaat 4860
ggccattttg catgtctctc tcccaggcct gctctcttga acagtgccga aaacttcact 4920
gtgctcatca agaacaatat cgacttcccc ggccacaact acaccacgta agtgcccagg 4980
ctgcctggct gtcttagtta tctactgctg agtaataaat tatcccaaac ctcagaagcc 5040
tgaaacaaca aacgcctatt gtctcccacg gtttctgtgg gtcaggaatc tgggaatgac 5100
tttgctgcgt ggttctggct caaggtctgt caggttgtag ccaagctgtc aaccagggct 5160
gcagtcattt ctaggcttga ctggggctgg agaacccttt tccaagctct cacacagttg 5220
ctcgtgggag agctcagttc ctcaccacgt gaacctcgcc ctagaccact tgagtatcct 5280
tggtatatgg tggctggctt ctcccagagc aagtgaccca agagagacag agcaagcaac 5340
caagagtata accaagatgg aagccacagt ctttgggggg agaccccaac acttctgcca 5400

tatgccattg gtcacacaga tcaaccctgg tccagtgtga gaggccactg cccaggggtc 5460
ccaggaggca gggatcattt ggggctttca tggaacctct ccaccacact ggctcactcc 5520
tgggaaagag acagatctgt tttcaatcga gatgtttgtt tgtttgcttt taattatgga 5580
caggagaaac atcctgccag gtttaaacat cacttgtacc ttccacaaga ctcagaatcc 5640
acagtgtccc attttccgac taggagacat cttccgagaa acaggcgata atttttcaga 5700
tgtggcaatt caggttggtg gtgctttgta cactgggatg tggggctgtg tgtctaggga 5760
tggaggatgt caaacagcca agaggccggg ccactgggtc ttcataatgt ggctcacatt 5820
tactgagcat ttagtaaatc cacccgctgc gctaaggtct ttacctacgc tacctcgtca 5880
aatcccaaaa caatccttat gagtgagagc tacttggtgt attcctttcc tgtggctgct 5940
gtagcaagtt atcaaagctt agtggcttca aacaacacat atttgcttat gttgccagag 6000
atcagaagtt ggagatgatt ttccctgagc cagggcggtg ctccctctgg gactttaagg 6060
gagaatccag ttcctcagct tttccacctt ctggagctgc attccttgca tttcttcaaa 6120
gccagcagca taacatcttg cctcagtggc cactttcact ccctatcctg tgtccaatct 6180
ccctttgcct ctgtcttaaa aagagagaga gcatttacaa gagggggcat ttaaggacca 6240
actggataat ccaggataat ctcccatctc aagatccttc atttaggctg ggcacggtgg 6300
ctcatgcctg taatcccagc actttgggag gctgaggtgg gtggatcacc tgaggtcagg 6360
agttcaacac cagcctggcc aacatggtga aagcccatct ttactaaaaa tacaaaaaaa 6420
aaaaaaaaat agccgggcat gattgcgggc tcctgtaatc ccagctactc gggaggctga 6480
gacaggagaa tcgcttgaac ctgggaggca gaggttgcag tgagccgaga tcgcaccact 6540
gcactccagc ctaggtgaca agagcgaaac tccatctcaa aaaaaaaaaa aaaatccttc 6600
atgtattcgc atctgcaaag agctttccct aggggagtac taggaggtaa agcagaaaag 6660
atatttgata gagtgccctg aattccagtc taataagttt ggacttgatc tttaatgggg 6720
gcgtgggggg cattaaaggt gtttgggtac aggagtggtc tgttgaaagt tgtattttag 6780
gacaatgagt ttaacagtga tgtgtcccag acgggggtag ggagagtgag gagatgcgat 6840
tgtggctgcc acaataacac ttgtgcgagt taggtggggc tgtacatatg gttcttcaat 6900
cagcattttt tctctaaaaa ccttaagcaa tcctggctat gcagggagat gtctggcggt 6960
tgcgtaactc acacccagca gccatagaga ctgtcccttg ttgatccttc agggcggaat 7020
aatgggcatt gagatctact gggactgcaa cctagaccgt tggttccatc actgccgtcc 7080
caaatacagt ttccgtcgcc ttgacgacaa gaccaccaac gtgtccttgt accctggcta 7140
caacttcagg taactccaag gcccaggtca aactcaccca gtggctgaat cgcattccca 7200
ggaactggtg agactaattt tggtttccaa ggcaacaaga tgaatgaaaa aagactttct 7260
ctaagaacta ggtgataact gaattttttc cataattttt taaaattctc aaaagagata 7320
cactctttat tttttactta tttttttttt tttgaaatgg agtctcactc tgtcacccag 7380
gctgaagtgc agtggcgcca tctcagtcac tgcaaacttc cgcctcccag gttcaagcga 7440
ctctcctgcc tcagcctccc aagtagctga gattacaggc ggatgcacac tgtttataaa 7500
acaaaactat tgggaaacag aaaagcatag agggggatca aaatcgccca taattcccct 7560
accctgaaat aatcaataac aaccctcggg ggaattttcc tcatctgtac caattatttc 7620
atacagctcc tatgagatca tagcatatat atatatatat cttgtggtat tctgcagggt 7680
ttttcatacc acagccactc aaaattcttt gtaaccatca cattaatgat cataacattc 7740
cattttgtag gtgaacaaat aacaactgct acaattcagg aagtgttttc ttttcttttc 7800
ttttcttttc tttttttttt ttagatggag tcacactctg cttgcccagg ctggagtgca 7860
gtggcatgat ctcagctcac tgcaacctct gcctcctagg tccaagcgat cctcccacct 7920
cccaagtttc tgggaccaca ggcatgtgcc accacaccca gctaattttt gtatattcag 7980
tagagatggg gtttcactgt gttggccagt ctggtctcga actcttgacc tcaagtgatc 8040
ttcccacctt ggcttcccaa agtgctagga ttacagtcat gagccactgt gcctggccca 8100
aggagggttt tccatatacc aagcactccc catcgccatc cctaaatctc ccaacaaccc 8160
tggaaggaag atattgtttc tggaagatga tttgcccaag acccacagct gatagtacat 8220
gtttgcataa ttctaaccca cgttcactct gaccccacac tcacactccc atcccttccc 8280

ttcccatctc aatgattttc tcaccgtacg cctccatgaa ttgaatattt gagttgcttc 8340
ccagtttttc tagtacaagt aaccacagtg tgcatctttg caccgtaaac ttcttctttg 8400
aattccaggg ttacttccct aggataaatt tcctagactt attgaatcaa aggttgtgaa 8460
cattttatca tatgcttttt atttttaaaa atatctatgg ttataatgtt tcattttttt 8520
ttctgagaca gagtctcact ctgtcaccca ggttggagtg gaccgggtgc aattatagct 8580
cactgcaacc tctgcctccc aggcccaagt gatcctccca cctcagcctc ctgagtagct 8640
aggactacag gtgcatgcca ccatgcccag ctaattttta aaattttttt gtagagtcgg 8700
ggtctcacta tattgcccag gctggtctca aactcctggc tcaagcaatc cgcctgcctt 8760
ggcctcctaa agcgttggga ttgcaggtgt gagctactgc acctggccta taattttcat 8820
ttctaggatt tttatttggt gctttttcaa agtcatctat tattgttcca gtgagtccca 8880
ttcttacctt aaggatccta ctccttctgt ccattctact gtatcattcc tttcataccg 8940
acctattatc tgaagtaact tgggtgggag ttctcctcgt gggctttgaa atactgtctt 9000
cagtagaaaa gatcttatgc aaagttctgc atgtgctgtg tgttatggag tattccttat 9060
atattactca aagcagcttc atgaactggc aggcagcccc ttaaatgtgt ttctgtttct 9120
gtttttttgt tttttggtct gcatgatgtt ttaaaacttg agacaggcca ggtgcggtgg 9180
cttatgcctg taatcccagc actttgggag gccaaggtgg gaggatcact tgaacccggg 9240
ggtttgaggc tagcctgggc aacataggga gaccccatct ctacaaataa taatttttaa 9300
aacatcagcc aggtgtgggg gcatgcacct ctggtcccag ctactcagga ggctgaggca 9360
ggaggattgc ttgaacctgg gaggttgagg ctgcagtgaa ccgtggttgt gccactgcac 9420
tccagcctgg gtgacagagt gagaccctta catggtggcc actggctgga gctgagtatc 9480
agtggtccta tttagaaagg ggctgggctt tctggttcat cactgtcccc gccgctcctt 9540
agtgcttata cctggcccac atcactcatt tctgtcatct gcctggcccc tgtgtagaca 9600
tttgagtttg aaacccttga ctcaaaggca ggctgatgct ttttgcttcc tctggatcaa 9660
tggaatttca tccaaggcaa tggaaccagc tcctaattgt gataactttt tgatcatccc 9720
ctgccactgt gctgaatagg cttatggcca ttaagaagag aggacaggat gaatggtccc 9780
ctgactgact gcccttccag ggtgtttttt tagctgtcag cagaagcatg cggggcagtg 9840
taccaatcag gtgtcagcaa gtgtccttcc agcgactgag ttgagcacaa aaacatctgc 9900
tcctggagag acctgagccc tctgaaggcc tcagccagtt atgatgttaa tgttctttta 9960
gaacaaagtg gtacacccat ttgttgtctg gaatgagcac cagaacaaat ctttggccaa 10020
aaataaattg ttgagggccg ggagcagtgg cttatgcctg taatcccaac actttgggag 10080
gccgaagcag gaggatcacc tgaggtcagg agttcgagac cagcctggcc aacatggtga 10140
aaccctgtct ctcctaaaaa tacaaaaatt agctggacgt ggtgacaggt gcctgtaata 10200
cagctactgg gaggctgagg cagggagaac tgcttgaacc cggaaggtgg aggttgcagt 10260
gagccggggt catgccactt cactccagcc tgggcaacag agcaaagctc tgtctttaaa 10320
aacaacttaa ataaataaat aaattgttga ggtctgatga gtaagtggac aagttatttt 10380
ccagcagaca cacaaaagag aaggaaatta caggttatag gaggtatttc agaaaatata 10440
actttctaaa acataggaag ttgaagaagt tgatcacatt acagaattct gttgtttaga 10500
aaatgacctg tggcgaaatg tccttattca gtgaataggt gattccgctt atgcacgacc 10560
tgtgtgaagt ggatcaggcc acccagaatg cacgatgcgc ttctcaggcc cagcaggagt 10620
atgtgtctgt gttaatttcc tgtggctatt atgactaatt gccacaaatg tggtggctta 10680
aaacaacaga aattaatctt cttatagttc tggaagccag aagtttggaa tcaagatgtc 10740
agcagggcca cactcgctct gatgctctac gggagggtcc tctcttgcct cttccagcgt 10800
ctggtggctc caggcattcc ataactttat agcagcgtcc cacaaatctc tgcctccatc 10860
ctcacatggc cttctccact gtgtctctat gtcttcaatc tctttttttt tttttttttt 10920
ttgaggcagg gtttcactcc agtctcctag actgaagtgc aatggcgtaa tttcgggtca 10980
ctgcaacctc tgcctcccgg gctcaagcga tttgatctct cctttatctt ataaagatac 11040
tagtcattgg atttggggct taccctaaat ccaggataat ctcatcttga gatgtttaac 11100
ttaattatat ctgcaaacac tgtatttcca aataaggtca tatcacagcc actagggatt 11160

agatacttga acatatctta tttgggggct caacccattc cagtgtacga aaaacactct 11220
tgttcaaggc ccgatgtttc tcagggcata gcccactgac tacctg 11266

<210> 4
<211> 595
<212> PRT
<213> Homo sapiens

<400> 4